(19) Europäisches Patentamt

European Patent Office

Office européen des brevets

(11) Veröffentlichungsnummer : **0 139 941**
**B1**

(12) **EUROPÄISCHE PATENTSCHRIFT**

(45) Veröffentlichungstag der Patentschrift :
23.03.88

(51) Int. Cl.⁴ : **G 06 F   3/037**, G 06 K 11/06

(21) Anmeldenummer : **84109579.7**

(22) Anmeldetag : **10.08.84**

(54) Röntgendiagnostikanlage mit einer Patientenlagerstatt und einer Primärstrahlenblende.

(30) Priorität : 24.08.83 DE 3330552

(43) Veröffentlichungstag der Anmeldung :
08.05.85 Patentblatt 85/19

(45) Bekanntmachung des Hinweises auf die Patenterteilung : 23.03.88 Patentblatt 88/12

(84) Benannte Vertragsstaaten :
DE FR

(56) Entgegenhaltungen :
EP-A- 0 021 928
DE-A- 2 613 809
DE-A- 2 655 661
GB-A- 2 096 867
US-A- 3 720 948
US-A- 3 978 280
US-A- 4 245 244

(73) Patentinhaber : Siemens Aktiengesellschaft Berlin und München
Wittelsbacherplatz 2
D-8000 München 2 (DE)

(72) Erfinder : Born, Hans-Joachim
Drosselweg 3
D-8521 Möhrendorf (DE)
Erfinder : Duschka, Hartmut
Amselweg 5
D-8525 Uttenreuth (DE)
Erfinder : Seyler, Gerhard
Lukasstrasse 6
D-8521 Bubenreuth (DE)
Erfinder : Zerl, Wolfgang
Sperberweg 3
D-8522 Herzogenaurach (DE)

## Beschreibung

Die Erfindung betrifft eine Röntgendiagnostikanlage mit einer über eine Steuerschaltung motorisch verstellbaren Patientenlagerstatt, einer motorisch verstellbaren Primärstrahlenblende und einer Bildverstärker-Fernsehkette zur Bildwiedergabe.

Röntgendiagnostikanlagen dieser Art dienen zur Erzeugung eines Röntgenfernsehbildes von einem Patienten. Zur Auswahl des jeweils durchstrahlten Bereiches für eine Röntgenaufnahme wird dabei die Patientenlagerstatt unter Durchleuchtungskontrolle in eine gewünschte Stellung verfahren, in der der für die Aufnahme wesentliche Teil des Patienten abgebildet wird. Ferner wird die Primärstrahlenblende den Abmessungen dieses Teiles entsprechend eingestellt. Demgemäß sind vor einer Röntgenaufnahme umständliche und zeitraubende Einstell- und Auswahlvorgänge erforderlich. Insbesondere ist es häufig erforderlich, Bedienvorgänge wechselweise durchzuführen, d. h. zunächst die Patientenlagerstatt durch entsprechende Motorsteuerung zu verstellen, dann die Blende zu betätigen, dann die Stellung der Patientenlagerstatt nachzukorrigieren usw.

In der DE-A-2 655 661 ist ein Computertomograph beschrieben, der eine Meßanordnung mit einer Röntgenröhre und einem Strahlenempfänger und eine relativ zu der Meßanordnung in Richtung ihrer Längsachse verschiebbare Patientenlagerstatt aufweist. Zur Positionierung der Patientenlagerstatt relativ zu der Meßanordnung sind eine zusätzliche Röntgenröhre und ein Röntgenbildverstärker mit nachgeschalteter Fernsehkette vorgesehen, die ein Durchleuchtungsbild des Patienten erzeugen, das auf dem Monitor der Fernsehkette dargestellt wird. Am Rand des Bildschirmes des Monitors sind Schaltmittel angeordnet, die bei ihrer Betätigung eine Verstellung der Patientenlagerstatt um ein solches Maß bewirken, daß die auf dem Bildschirm ihnen gegenüberliegend abgebildete Körperschicht in den Strahlengang der Meßanordnung gefahren wird. Es sind also zwei Röntgensysteme vorhanden, wobei das Bild des einen zur Positionierung der Patientenlagerstatt in bezug auf das andere Röntgensystem dient. Für eine gewöhnliche Röntgendiagnostikanlage ist eine solche Lösung nicht vorteilhaft, da infolge des Umstandes, daß eine zweite Röntgenröhre und ein zweiter Röntgenbildverstärker mit Röntgenfernsehkette erforderlich sind, eine entsprechende Röntgendiagnostikanlage unwirtschaftlich und außerdem unnötig kompliziert wäre. Außerdem ist eine Positionierung der Patientenlagerstatt nur in deren Längsrichtung möglich, was zwar für einen Computertomographen, mittels dessen ausschließlich quer zur Längsachse des Patienten verlaufende Schichten aufgenommen werden, nicht jedoch für eine gewöhliche Röntgendiagnostikanlage zur Anfertigung von Röntgenschattenbildern ausreicht.

Der Erfindung liegt deshalb die Aufgabe zugrunde, eine Röntgendiagnostikanlage der eingangs genannten Art so auszubilden, daß die Einstellvorgänge, die erforderlich sind, um eine in einem auf dem Monitor dargestellten Durchleuchtungsbild sichtbare Region im Bildzentrum einer nachfolgenden Aufnahme, insbesondere einer Indirektaufnahme von dem Monitor, mittels des auch zur Erzeugung des Durchleuchtungsbildes dienenden Röntgensystems abzubilden, auf ein Minimum reduziert sind.

Diese Aufgabe ist erfindungsgemäß dadurch gelöst, daß dem Sichtgerät der Bildverstärker-Fernsehkette ein Lichtgriffel zur Eingabe bestimmter Stellen auf dem Sichtgerät in einen Speicher zugeordnet ist und daß an dem Speicher die Steuerschaltung für die Motoren der Patientenlagerstatt angeschlossen ist, die so ausgebildet ist, daß die Patientenlagerstatt entsprechend der jeweiligen Stelle auf dem Sichtgerät, auf die der Lichtgriffel ausgerichtet ist, im von der Primärstrahlenblende ausgehenden Strahlenbündel zentriert wird.

Bei der erfindungsgemäßen Röntgendiagnostikanlage genügt es zur Einstellung der Patientenlagerstatt anhand des jeweils auf dem Monitor sichtbaren Bildes die im Zentrum der nachfolgenden Aufnahme gewünschte Region mit Hilfe des Lichtgriffels zu markieren. Die Patientenlagerstatt wird anschließend automatisch motorisch so verstellt, daß die markierte Region im Zentrum des Bildes liegt.

Eine besonders zweckmäßige Weiterbildung der Erfindung besteht darin, daß eine Blendensteuerschaltung zur Steuerung der Primärstrahlenblende entsprechend dem jeweils durch den Lichtgriffel ausgewählten Bildfeld vorhanden ist. Bei dieser Weiterbildung wird durch die Markierung der jeweils interessierenden Region auf dem Sichtgerät nicht nur die Patientenlagerstatt in die richtige Position verfahren, sondern auch die Primärstrahlenblende automatisch motorisch so verstellt, daß die gewünschte Region eingeblendet wird.

Die Erfindung ist nachfolgend anhand eines in der Zeichnung dargestellten Ausführungsbeispieles näher erläutert. Es zeigen :

Fig. 1 eine Röntgendiagnostikanlage zur Erläuterung des Erfindungsgedankens,

Fig. 2 eine für die Erfindung wesentliche Einzelheit der Röntgendiagnostikanlage gemäß Figur 1, und

Fig. 3 eine Einzelheit aus der Figur 2.

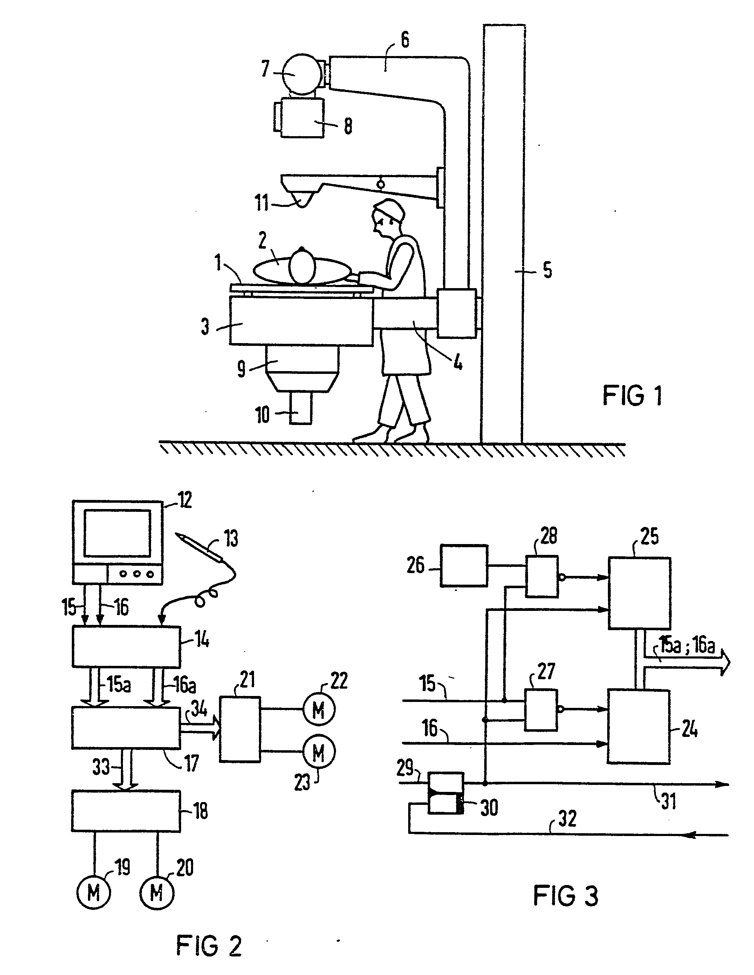

In der Figur 1 ist eine Patientenlagerstatt 1 dargestellt, auf der ein Patient 2 ruht, von dem Röntgenaufnahmen angefertigt werden sollen. Die Patientenlagerstatt 1 ist auf einem Rahmen 3 motorisch verschiebbar. Der Rahmen 3 ist an einem Träger 4 befestigt, der an einem Stativ 5 höhenverstellbar gelagert ist. Der Träger 4 trägt einen Arm 6 mit einer Röntgenröhre 7, die die den Patienten 2 durchdringende Röntgenstrahlung aussendet. Diese Röntgenstrahlung wird durch eine motorisch verstellbare Primärstrahlenblende 8 auf den gewünschten Bereich eingeblendet. Das Röntgenbild wird durch einen Röntgenbildverstärker 9 erfaßt, dem eine Fernsehkamera 10 nachgeschaltet ist. Ein Kompressionstubus 11 dient zur Kompression des Patienten 2 und ist hierzu am Arm 6 höhenverstellbar gelagert.

Das von der Fernsehkamera 10 aufgenommene Bild wird auf einem Sichtgerät 12 (Fig. 2) wiedergegeben, dem zur Markierung der jeweils interessierenden Region ein Lichtgriffel 13 zugeordnet ist. Zum Einstellen der Patientenlagerstatt 1 und der Primärstrahlenblende 8 mit Hilfe von Elektromotoren wird die jeweils interessante Region mit Hilfe des Lichtgriffels 13 markiert und die Komponenten 1 und 8 werden anschließend automatisch so verstellt, daß die markierte Region in der Mitte auf dem Sichtgerät 12 erscheint und optimal eingeblendet ist, so daß eine Überstrahlung des wiedergegebenen Bildes vermieden ist. Hierzu ist der Lichtgriffel 13 an einer Zählerschaltung 14 angeschlossen, der über eine Leitung 15 die Horizontalimpulse und über eine Leitung 16 die Vertikalimpulse aus dem Sichtgerät 12 zugeführt werden. Wird der Lichtgriffel 13 auf eine bestimmte Stelle des Bildes auf dem Sichtgerät 12 gesetzt, so sind die Koordinaten dieser Stelle in der in Verbindung mit der Figur 3 nachfolgend noch näher beschriebenen Weise aus der Zählerschaltung 14, wo sie vorübergehend gespeichert werden, abfragbar. Die x-Koordinate auf dem Sichtgerät 12 wird dabei durch die Datenleitung 15a und die y-Koordinate durch die Datenleitung 16a einem Mikrocomputer 17 übermittelt, der über eine Motorsteuerschaltung 18 die beiden Motoren 19 und 20 für die Längs- und Querverschiebung der Patientenlagerstatt 1 so ansteuert, daß diese die markierte Stelle ins Zentrum des Bildes auf dem Sichtgerät 12 rückt.

Der Mikrocomputer 17 bewirkt ferner über eine Blendensteuerschaltung 21 eine Ansteuerung der Motoren 22 und 23 für die Verstellung der Primärstrahlenblende 8, und zwar des Blendenplattenpaares für die Einblendung in x-Richtung und des Blendenplattenpaares für die Einblendung in y-Richtung entsprechend.

Es ergibt sich demgemäß, daß durch Markierung der relevanten Region auf dem Sichtgerät 12 mit Hilfe des Lichtgriffels 13 automatisch zugleich eine optimale Einstellung der Patientenlagerstatt 1 und der Primärstrahlenblende 8 erfolgen. Weitere Einstellhandhabungen sind hierzu nicht erforderlich.

Die Figur 3 zeigt den Aufbau der Zählerschaltung 14 für die Ermittlung derjenigen Stelle auf dem Sichtgerät 12, auf die der Lichtgriffel 13 ausgerichtet ist. Die Zählerschaltung 14 weist einen ersten Zähler 24 für die Horizontalimpulse auf der Leitung 15 und einen zweiten Zähler 25 für die Taktimpulse eines Taktgenerators 26 auf. Die Taktfrequenz des Taktgenerators 26 ist so gewählt, daß seine Impulse jede Bildzeile entsprechend einer gewünschten Zeilenauflösung unterteilen. Der erste Zähler 24 ist durch jeden Vertikalimpuls auf der Leitung 16 und der zweite Zähler

25 durch jeden Horizontalimpuls auf der Leitung 15 rückstellbar. Die Ansteuerung der Zähler 24 und 25 erfolgt über Gatter 27 und 28. Der jeweilige Zählerstand kann durch einen Impuls auf der Leitung 29, der die Zähler 24 und 25 über ein Flip-Flop 30 ansteuert und vom Lichtgriffel 13 geliefert wird, festgehalten werden.

Ist der Lichtgriffel 13 nicht auf das Sichtgerät 12 ausgerichtet, so werden die Zähler 24 und 25 der Bewegung des Elektronenstrahles des Sichtgerätes 12 entsprechend weitergeschaltet, d. h. der Stand des Zählers 24 entspricht der jeweils abgetasteten Bildzeile, da dieser Zähler 24 die Horizontalimpulse zählt und durch jeden Vertikalimpuls auf Null zurückgestellt wird. Der Stand des Zählers 25 entspricht der vom Elektronenstrahl in der jeweiligen Zeile erreichten Stelle, da dieser Zähler 25 durch jeden Horizontalimpuls auf Null gestellt wird und die Impulse des Taktgenerators 26 für jede Bildzeile zählt.

Wird der Lichtgriffel 13 auf eine bestimmte Stelle des Sichtgerätes 12 aufgesetzt, so wird auf der Leitung 29 ein Impuls erzeugt, der über das Flip-Flop 30 die Zählerstände der Zähler 24 und 25 festhält. Gleichzeitig wird über die Leitung 31 ein Interrupt-Impuls an den Mikrocomputer 17 gegeben, der daraufhin die Zählerstände über die Datenleitungen 15a und 16a abfragt. Ist diese Abfrage erfolgt, so liefert der Mikrocomputer 17 über die Steuerleitung 32 einen Freigabeimpuls, so daß die Zähler 24 und 25 wieder zu zählen beginnen und für die Erfassung der nächsten Stelle auf dem Sichtgerät 12, auf die der Lichtgriffel 13 ausgerichtet wird, bereitstehen. Der Mikrocomputer 17 bewirkt nun seinerseits über die Leitungen 33 und 34 (Fig. 2) die Einstellung der Patientenlagerstatt 1 und der Primärstrahlenblende 8.

**Patentansprüche**

1. Röntgendiagnostikanlage mit einer über eine Steuerschaltung (18) motorisch verstellbaren Patientenlagerstatt (1), einer motorisch verstellbaren Primärstrahlenblende (8) und einer Bildverstärker-Fernsehkette (9, 10, 12) zur Bildwiedergabe, dadurch gekennzeichnet, daß dem Sichtgerät (12) der Bildverstärker-Fernsehkette (9, 10, 12) ein Lichtgriffel (13) zur Eingabe bestimmter Stellen auf dem Sichtgerät (12) in einen Speicher (14) zugeordnet ist und daß an dem Speicher (14) die Steuerschaltung (18) für die Motoren (19, 20) der Patientenlagerstatt (1) angeschlossen ist, die so ausgebildet ist, daß die Patientenlagerstatt (1) entsprechend der jeweiligen Stelle auf dem Sichtgerät (12), auf die der Lichtgriffel (13) ausgerichtet ist, im von der Primärstrahlenblende (8) ausgehenden Strahlenbündel zentriert wird.

2. Röntgendiagnostikanlage nach Anspruch 1, dadurch gekennzeichnet, daß der Speicher (14) von zwei Zählern (24, 25) gebildet ist, von denen der erste (24) die Horizontalimpulse und der zweite (25) die Impulse eines Taktgenerators (26) zählt, dessen Taktfrequenz so gewählt ist, daß seine Impulse jede Bildzeile entsprechend einer gewünschten Zeilenauflösung unterteilen, daß der erste Zähler (24) durch jeden Vertikalimpuls und der zweite Zähler (25) durch jeden Horizontalimpuls rückstellbar ist, daß der Lichtgriffel (13) an beiden Zählern (24, 25) zum Festhalten des Zählerstandes beim Erreichen der jeweiligen Aufsetzstelle des Lichtgriffels (13) auf dem Sichtgerät (12) durch den Elektronenstrahl angeschlossen ist und daß die Zählerstände einem Mikrocomputer (17) zuführbar sind, der eine Motorsteuerschaltung (18) ansteuert.

3. Röntgendiagnostikanlage nach Anspruch 1 oder 2, dadurch gekennzeichnet, daß eine Blendensteuerschaltung (21) zur Steuerung der Primärstrahlenblende (8) entsprechend dem jeweils durch den Lichtgriffel (13) ausgewählten Bildfeld vorhanden ist.

**Claims**

1. An X-ray diagnosis system with a motorised patient couch (1) adjustable through a control circuit (18), a motorised adjustable primary radiation diaphragm (8) and an image intensifying television link (9, 10, 12) for the image reproduction, characterised in that a light pen (13), for entering particular positions on the display unit (12) into a store (14) is associated with the display unit (12) of the image intensifying television link (9, 10, 12) and that the control circuit (18) for the motors (19, 20) of the patient couch (1) is connected to the store (14) and is designed so that the patient couch (1) is centred in the beam of radiation from the primary radiation diaphragm (8) according to the respective position on the display unit (12) with which the light pen (13) is aligned.

2. An X-ray diagnosis system according to claim 1, characterised in that the receiving unit (14) comprises two counters (24, 25) of which the first (24) counts the horizontal pulses and the second (25) the pulses of a timing generator (26) of which the timing frequency is chosen so that its pulses subdivide each scanning line according to a desired line resolution, that the first counter (24) is resettable by each vertical pulse and the second counter (25) by each horizontal pulse, and that the light pen (13) is connected to both of the counters (24, 25) by the electron beam in order to retain the counter reading on reaching the respective aim point of the light pen (13) on the display unit (12), and that the counter readings can be supplied to a microcomputer (17) which directs a motor control circuit (18).

3. An X-ray diagnosis system according to claim 1 or claim 2, characterised in that there is an aperture control circuit (21) for controlling the primary radiation diaphragm (8) corresponding to the image field selected by the light pen (13).

**Revendications**

1. Installation de radiodiagnostic comportant un plateau formant couchette pour patient (1) déplaçable au moyen d'un moteur par l'intermédiaire d'un circuit de commande (18), un diaphragme du rayonnement primaire (8) déplaçable à l'aide d'un moteur, une chaîne de télévision à amplificateur de brillance (9, 10, 12) pour la reproduction des images, caractérisée le fait que l'appareil de visualisation (12) de la chaîne de télévision à amplificateur de brillance (9, 10, 12) est associé à un photostyle (13) servant à introduire des zones d'image déterminées sur l'appareil de visualisation (12) dans une mémoire (14) et que la mémoire (14) est raccordée au circuit (18) de commande des moteurs (19, 20) du plateau formant couchette pour patient (1), circuit qui est agencé de telle sorte que le plateau formant couchette pour patient (1) est centré dans le faisceau de rayonnement émanant du diaphragme de rayonnement primaire (8), conformément à la zone d'image respective située sur l'appareil de visualisation (10) et sur laquelle le photostyle (13) est dirigé.

2. Installation de radiodiagnostic suivant la revendication 1, caractérisée par le fait que la mémoire (14) est formée par deux compteurs (24, 25), dont le premier (24) compte les impulsions horizontales et dont le second (25) compte les impulsions d'un générateur de cadence (26), dont la fréquence d'horloge est choisie de manière que ses impulsions subdivisent chaque ligne de l'image conformément à une résolution de lignes désirée, que le premier compteur (24) peut être rétrogradé par chaque impulsion verticale et que le second compteur (25) peut être rétrogradé par chaque impulsion horizontale, que le photostyle (13) est raccordé aux deux compteurs (24, 25) pour la conservation de l'état de comptage lorsque le faisceau d'électrons atteint la position respective d'application du photostyle (13) sur l'appareil de visualisation (12), et que les états de comptage sont envoyés à un micro-ordinateur (17), qui commande un circuit (18) de commande des moteurs.

3. Installation de radiodiagnostic suivant la revendication 2, caractérisée par le fait qu'il est prévu un circuit (21) de commande du diaphragme, servant à commander le diaphragme (8) du rayonnement primaire, conformément à la zone d'image respectivement sélectionnée par le photostyle (13).

FIG 1

FIG 2

FIG 3